# EUROPEAN PATENT SPECIFICATION

(11) **EP 1 180 033 B1**
(45) Date of publication and mention of the grant of the patent: **11.02.2004**
(21) Application number: 00917372.5
(22) Date of filing: 20.04.2000
(51) Int. Cl.: A61K 31/506, A61K 9/16, A61P 31/12, A61K 47/38

(54) **LAMIVUDINE CONTAINING PHARMACEUTICAL FORMULATION**
LAMIVUDIN ENTHALTENDE ARZNEIMITTEL
FORMULATION PHARMACEUTIQUE CONTENANT DE LA LAMIVUDINE

(30) Priority: 22.04.1999 GB 9909154
(43) Date of publication of application: 20.02.2002
(73) Proprietor: Glaxosmithkline Kabushiki Kaisha, Tokyo (JP)
(72) Inventor: KAWAMURA, Koho, Imaichi-shi, Tochigi 321-1274 (JP); MARUYAMA, Toshio, Imaichi-shi, Tochigi 321-1274 (JP); MISHIMA, Yasuhiro, Imaichi-shi, Tochigi 321-1274 (JP); SUGIBAYASHI, Nobuya, Imaichi-shi, Tochigi 321-1274 (JP)
(74) Representative: Giddings, Peter John, Dr.
(86) International application number: PCT/JP2000/002572
(87) International publication number: WO 2000/064427

(56) References cited:
- EP-A- 0 288 138
- WO-A-91/17159
- WO-A-97/06804

## Description

The present invention relates to a pharmaceutical formulation of lamivudine useful in the treatment of disease in mammals, including humans. The invention also relates to the preparation of such a formulation.

The present invention relates to a pharmaceutical formulation of lamivudine useful for the treatment of viral infections, particularly hepatitis B virus infection (HBV) and human immunodeficiency virus (HIV). Lamivudine has the chemical name, (2R,cis)-4-amino-1-(2-hydroxyrnethyl-1,3-oxathiolan-5-yl)-pyrimidin-2-one.

Hepatitis B is a viral disease transmitted orally or parentally by contaminated material such as blood or blood products, contaminated needles, sexually, and vertically from infected or carrier mothers to their off-spring. In those areas of the world where the disease is common, vertical transmission at an early age results in a high proportion of infected individuals becoming chronic carriers of hepatitis B. An estimated 350 million people world-wide are chronically infected with hepatitis B and as many as 150 million may die from liver disease in the absence of intervention.

Currently, the only established approach to the treatment of hepatitis B infections is repeated injections of interferon, which may be associated with unpleasant side effects, and produces a long lasting response in only one third or less of those treated. Interferon is an immune modulator designed to boost the disease fighting ability of the immune system.

Multiple clinical trials have established that lamivudine has an excellent efficacy and safety profile across diverse patient groups from Asia, Europe and North America. Furthermore seroconversion is achieved by up to 65% of patients with greater than twice the upper limit of the normal level of a liver enzyme called ALT, after three years of treatment with lamivudine. Lamivudine is currently on sale in many countries including the United States of America and Europe for this indication under the trademark ZEFFIX™.

A species of retrovirus, the human immunodeficiency virus (HIV) has been reproducibly isolated from patients with AIDS (acquired immunodeficiency syndrome) or with the symptoms that frequently precede AIDS. AIDS is an immunosuppressive or immunodestructive disease that predisposes subjects to fatal opportunistic infections. Characteristically, AIDS is associated with a progressive depletion of T-cells, especially the helper-inducer subset bearing the CD4 surface marker. HIV is cytopathic and appears to preferentially infect and destroy T-cells bearing the CD4 marker, and it is now generally recognised that HIV is the etiological agent of AIDS. Clinical conditions such as AIDS-related complex (ARC), progressive generalised lymphadenopathy (PGL), Karposi's sarcoma, thrombocytopenic purpura, AIDS-related neurological conditions, such as AIDS dementia complex, multiple sclerosis or tropical paraparesis, and also anti-HIV antibody-positive and HIV-positive conditions, including such conditions in asymptomatic patients, are also conditions which may be treated by appropriate anti-viral therapy. Lamivudine has proven antiviral activity against HIV and is currently on sale in many countries including the United States of America and Europe for this indication under the trademark EPIVIR®.

Lamivudine can be used in combination with a second antiviral agent for example known inhibitors of HIV or HBV replication. Such inhibitors include for example those which inhibit HIV reverse transcriptase HIV protease and TAT. Such inhibitors include for example, zidovudine (AZT), zalcitabine (ddC), didanosine (ddl), stavudine (D4T), delavirdine, nevirapine, (1 S,4R)-cis-4-[2-amino-6-(cyclopropylamino)-9H-purin-9-yl]-2-cyclopentene-1-methanol (abacavir, 1592U89), 3S-[3R*(1R*,2S*)]-[3-[[(4-aminophenyl)sulphonyl](2-methylpropyl)-amino]-2-hydroxy-1-phenylmethyl)propyl]carbamic acid, tetrahydro-3-furanyl ester (141 W94, amprenavir), efavirenz, tenofovir disoproxil (PMPA), (-)-beta-D-2,6-diaminopurine dioxolane (DAPD), adefovir dipivoxil (bis-Pom PMEA).

Preferably lamivudine is used in combination with zidovudine or abacavir, most preferably zidovudine.

For the treatment of HBV, lamivudine may be used in combination with adefovir dipivoxil.

Lamivudine, methods for its preparation and dosages are disclosed in inter alia, WO91/17159 and WO95/28174. Lamivudine, in both tablet form and oral solution, has been granted marketing approval in a number of countries. WO91/17159 generically discloses formulations for oral administration including granules, however no formulations for oral granules are specifically disclosed therein.

We have now found that lamivudine can be formulated as oral granules suitable for reconstituting in the Pharmacy which can then be dose titrated by body weight for patients, for example for children.

Oral granules are preferred in certain countries as they have advantages associated with tablets i.e. they are easy to store and stable over long periods but oral granules represent an advantage over tablets as the dose can be manipulated depending on the body weight of the patient. They are also more easily administered for those patients who have difficulty swallowing tablets. Oral granules can be administered either by simply swallowing of the granules, or dissolution in an aqueous solution which is then administered to the patient e.g. granules are particularly convienent for oral administration to children.

We have now found that the formulations of the present invention are particularly suitable for formulating oral granules.

The present invention provides a pharmaceutical formulation comprising:
(a) from 1 to 50% by weight of lamivudine, or a pharmaceutically acceptable derivative thereof;
(b) from 20 to 70% by weight of a bulking agent;
(c) from 20 to 50% by weight of a component which is both a bulking agent and a binder;
(d) from 1 to 20% by weight of a binder; and
(e) from 1 to 10% by weight of a lubricant.

Formulations of the present invention have the following properties:
(i) at least 90% by weight of the granules have a particle size of from 75 to 850 µm
(ii) 80% of the granules dissolve within 15 minutes, according to the Dissolution Test of The Pharmacopoeia of Japan, Thirteenth Edition 1996.

Preferably no granules have a particle size greater than 850 µm.

Preferably the pharmaceutical formulation comprises:
(a') from 1 to 50% by weight of lamivudine, or a pharmaceutically acceptable derivative thereof;
(b-1') from 20 to 50% by weight of lactose;
(b-2') from 1 to 20% by weight of crystalline cellulose;
(c') from 20 to 50% by weight of starch;
(d') from 1 to 20% by weight of hydroxypropyl cellulose; and
(e') from 1 to 10% by weight of talc.

One aspect of the invention is the use of a pharmaceutical formulation according to the invention in the manufacture of a medicament for use in the treatment of mammal including humans suffering from viral infections. A further aspect is the use of lamivudine for the manufacture of a pharmaceutical formulation according to the invention for the treatment of viral infections.

Lamivudine is provided on particles of lactose which act as a bulking agent. Lactose is a cheap material. Starch has a combined function. It acts as a bulking agent (diluent) and a binder and furthermore it also acts as a disintergrant when the granules are dissolved. Crystalline cellulose acts as a bulking agent and improves the granulation, fluidity of the formulation and it prevents particle adhesion. Hydroxypropyl cellulose acts as a binder. The talc acts to improve fluidity of the formulation and prevent adhesion of the granules.

As used herein "pharmaceutically acceptable derivative" includes any pharmaceutically acceptable salt, ester or salt of such ester of lamivudine which, upon administration to the recipient, is capable of providing (directly or indirectly) lamivudine or an antivirally active metabolite or residue thereof.

Pharmaceutically acceptable salts of lamivudine include those derived from pharmaceutically acceptable bases, inorganic and organic acids. Examples of suitable acids include hydrochloric, hydrobromic, sulphuric, nitric, perchloric, fumaric, maleic, phosphoric, glycollic, lactic, salicylic, succinic, toluene-p-sulphonic, tartaric, acetic, citric, methanesulphonic, formic, benzoic, malonic, naphthalene-2-sulphonic and benzenesulphonic acids. Other acids such as oxalic acid, while not in themselves pharmaceutically acceptable may be useful in the preparation of salts useful as intermediates in obtaining lamivudine and its pharmaceutically acceptable acid addition salts. Salts derived from appropriate bases include alkali metal (e.g. sodium), alkaline earth metal (e.g. magnesium), ammonium and NR₄₊ (where R is C₁₋₄ alkyl) salts.

To meet the standard on fine granules defined in the Japanese Pharmacopoeia, at least 90% by weight of the granules have a particle size of from 75 to 850µm, for example, from 92 to 95% by weight of the granules. Preferably up to 98% by weight of the granules of the invention have a particle size of from 75 to 850µm. Preferably no more than 5% by weight of the granules have a particle size greater than 500µm, for example, no more than 3% by weight. Desirably, less than 0.5% of the granules have a particle size greater than 500 µm. Particle size is determined by the Particle Size Distribution Test for Powders, The Pharmacopoeia of Japan, Thirteenth Edition 1996.

Typically, at least 80% of lamivudine or pharmaceutically acceptable derivative thereof in the formulation may be dissolved within 15 minutes according to the Dissolution Test, method 2 (paddle method). The amount of lamivudine dissolved may be determined by an appropriate physicochemical technique, for example, by ultraviolet (UV) analysis or by high pressure liquid chromatography (Nisso HPLC).

The formulation of the invention may be generally dust-free. It is preferably white although it may be white to off-white. The formulation is free-flowing, as may be determined by the eye. Typically the bulk density is from 0.4 to 0.7 g/cm³, for example, from 0.4 to 0.6 g/cm³, preferably 0.5g/cm³. Residual moisture levels are generally less than 10% by weight, for example, less than 5% by weight.

Preferred formulations contain from 5 to 15% by weight of lamivudine or pharmaceutically acceptable derivative. A particularly preferred formulation contains 10% by weight of lamivudine or pharmaceutically acceptable derivative. The amount of lactose and starch in the formulations are greater than the smaller amount of lamivudine or pharmaceutically acceptable derivative which is present. Suitable amounts of lactose and starch may each be from 32 to 42% by weight, preferably 37 % by weight of formulation.

The powders of the invention may contain from 1 to 10% by weight, of crystalline cellulose. Powders containing 5% by weight of crystalline cellulose are preferred.

Preferably the amount of hydroxypropyl cellulose present is from 3 to 13% by weight, of the formulation. Powders containing 8% by weight of hydroxypropyl cellulose are preferred.

Preferably the amount of talc present is from 1 to 5% by weight of the formulation, such as 3% by weight.

Even more preferably the formulation comprises:
(a") from 5 to 15% by weight of lamivudine or a pharmaceutically acceptable derivative;
(b-1 ") from 32 to 42% by weight of lactose;
(b-2")from 1 to 10% by weight of crystalline cellulose;
(c") from 32 to 42% by weight of starch;
(d") from 3 to 13 by weight of hydroxypropyl cellulose; and
(e") from 1 to 5% by weight of talc.

An especially preferred formulation comprises 10% by weight of lamivudine, 37% by weight of each of lactose and starch, 5% by weight of crystalline cellulose, 8% by weight of hydroxypropyl cellulose and 3% by weight of talc.

Additionally there is provided a pharmaceutical formulation comprising:
(a) from 1 to 50% by weight of lamivudine, or a pharmaceutically acceptable derivative thereof and a second antiviral agent;
(b) from 20 to 70% by weight of a bulking agent;
(c) from 20 to 50% by weight of a component which is both a bulking agent and a binder;
(d) from 1 to 20% by weight of a binder; and
(e) from 1 to 10% by weight of a lubricant.

The formulation of the invention is provided by a process which comprises at least two steps
(i) aqueous granulation of components (a) to (d) of the formulation; and:
(ii) blending the granules formed by step (i) with component (e) of the formulation.

Aqueous granulation is technique known in the art, for example *Remmington Pharmaceuticals 17*^{*th*} *Edition page 1610*.

A further aspect of the invention is granules comprising;
(a) from 1 to 50% by weight of lamivudine, or a pharmaceutically acceptable derivative thereof;
(b) from 20 to 70% by weight of a bulking agent;
(c) from 20 to 50% by weight of a component which is both a bulking agent and a binder; and
(d) from 1 to 20% by weight of a binder.

The granules can be made by the process of aqueous granulation of components (a) to (d) of the formulation.

Preferably the granules comprise;
(a') from 1 to 50% by weight of lamivudine, or a pharmaceutically acceptable derivative thereof;
(b-1') from 20 to 50% by weight of lactose;
(b-2') from 1 to 20% by weight of crystalline cellulose;
(c') from 20 to 50% by weight of starch; and
(d') from 1 to 20% by weight of hydroxypropyl cellulose.

Even more preferably said granules comprise;
(a") from 5 to 15% by weight of lamivudine or a pharmaceutically acceptable derivative;
(b-1 ") from 32 to 42% by weight of lactose;
(b-2")from 1 to 10% by weight of crystalline cellulose;
(c") from 32 to 42% by weight of starch; and
(d") from 3 to 13 by weight of hydroxypropyl cellulose.

Especially preferred the granules comprise 10.3% by weight of lamivudine, 38.1% by weight of each of lactose and starch, 5.2% by weight of crystalline cellulose, 8.3% by weight of hydroxypropyl cellulose by weight of the granules.

Additionally there is provided granules comprising:
(a) from 1 to 50% by weight of lamivudine, or a pharmaceutically acceptable derivative thereof and a second antiviral agent;
(b) from 20 to 70% by weight of a bulking agent;
(c) from 20 to 50% by weight of a component which is both a bulking agent and a binder; and
(d) from 1 to 20% by weight of a binder.

A formulation of the invention may be prepared by a process which comprises granulating the lamivudine or a pharmaceutically acceptable derivative, lactose, starch, crystalline cellulose and hydroxypropyl cellulose in the presence of, water. Lamivudine, lactose, starch, crystalline cellulose, hydroxypropyl cellulose may each be provided as powders having particle sizes, for example, average particle sizes, below 850µm and, indeed, below 200µm. These five components may be blended by a rotating fluid bed granulator while water is sprayed into the granulator. The resultant blend is dried. The dried granules are removed from the granulator and subjected to sieving. Any granules remaining on the sieve are then ground.

The resultant granules are mixed with talc by a V-shaped mixer.

Lamivudine or the pharmaceutically acceptable derivative employed as a starting material typically has a particle size of 200 µm or less. The starting lactose generally has a particle size of below 250µm, especially 150µm or less such as from 50 to 150µm. Lactose may be Pharmatose 200M.

Starch may be rice, wheat, potato or corn starch. Corn starch is alternatively termed maize starch and is preferred. A powder of starch of a particle size of from 30 to 150 µm is typically used as a starting material. Preferably starch is a partially pregelatinised starch such as Starch 1500G (Colorcon).

Crystalline cellulose typically is a powder of, for example, an average particle size of from 40 to 100 µm such as from 50 to 90 µm. A suitable crystalline cellulose is Avicel PH 102 having an average particle size of 80 µm. Crystalline cellulose is alternatively called microcrystalline cellulose.

Hydroxypropyl cellulose will be in powder of for example, an average particle size of from less than 710 µm, preferably at least 95% of the powder having a particle size of less than 500µm. A suitable hydroxypropyl cellulose is HPC-L (Nippon Soda).

Talc is a purified, hydrated, magnesium silicate, approximating to the formula Mg₆(Si₂O₅)₄(OH)₄. A suitable talc is CROWN TALC KYOKU PP (Matsumura Sangyo).

The formulation produced by aqueous granulation and subsequent mixing with talc is then introduced into a container which is then closed. The container may be sealed. It may be a single-dose or multi-dose container. The container may be a jar, bag or sachet. Sachets, especially foil sachets, are particularly suitable.

The following example illustrates the invention.

### Example 1

500g of the formulation was prepared by aqueous granulation.

### Formula I

| | |
|---|---|
| Lamivudine | 10% by weight |
| Lactose (Pharmatose 200m) | 37% by weight |
| Partly pregelatinized starch (Starch 1500G) | 37% by weight |
| Microcrystalline cellulose (Avicel PH-102) | 5% by weight |
| Hydroxypropyl cellulose (HPC-L) | 8% by weight |
| Talc (Crown Talc) | 3% by weight |

1. Lamivudine, lactose, partly pregelatinized starch, microcrystalline cellulose and hydroxypropyl cellulose were blended by a rotating fluid granulator while water was sprayed into the granulator.
2. The resultant blend was dried at a temperature of 75°C for 30 minutes.
3. The dried granules were then removed from the granulator and sieved through a 500 µm sieve to remove the over-sized component of the granules.
   The oversized components were then ground, and added to the remaining granules.
4. The resultant granule mixture was blended for 30 minutes with talc in a V-shaped mixer to produce fine granules.

The properties of the fine granules obtained are as follows.

| | |
|---|---|
| Yield % | **93** |
| Water content % | 2.7 |
| Bulk Density g/cm³ | 0.5 |
| Fluidity as expressed by angled repose° | 40 |
| % by weight of granules over 850µm in size | 0 |
| % by weight of granules over 500 µm in size | 0.3 |
| % by weight of granules over 75 µm in size | 92.9 |
| Dissolution test* | complies |

| | |
|---|---|
| * Dissolution Test, The Pharmacopoeia of Japan, Thirteenth Edition 1996. | |

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

## Claims

1. A pharmaceutical formulation comprising:
(a) from 1 to 50% by weight of lamivudine, or a pharmaceutically acceptable derivative thereof;
(b) from 20 to 70% by weight of a bulking agent;
(c) from 20 to 50% by weight of a component which is both a bulking agent and a binder.
(d) from 1 to 20% by weight of a binder; and
(e) from 1 to 10% by weight of a lubricant.

2. A pharmaceutical formulation as claimed in claim 1 comprising:
(a') from 1 to 50% by weight of lamivudine, or a pharmaceutically acceptable derivative thereof;
(b-1') from 20 to 50% by weight of lactose;
(b-2') from 1 to 20% by weight of crystalline cellulose;
(c') from 20 to 50% by weight of starch;
(d') from 1 to 20% by weight of hydroxypropyl cellulose; and
(e') from 1 to 10% by weight of talc.

3. A pharmaceutical formulation as claimed in claim 1 or claim 2 comprising:
(a") from 5 to 15% by weight of lamivudine or a pharmaceutically acceptable derivative;
(b-1") from 32 to 42% by weight of lactose;
(b-2") from 1 to 10% by weight of crystalline cellulose;
(c") from 32 to 42% by weight of starch;
(d") from 3 to 13 by weight of hydroxypropyl cellulose; and
(e") from 1 to 5% by weight of talc.

4. A pharmaceutically acceptable formulation comprising 10% by weight of lamivudine, 37% by weight of each of lactose and starch, 5% by weight of crystalline cellulose, 8% by weight of hydroxypropyl cellulose and 3% by weight of talc.

5. Granules comprising:
(a) from 1 to 50% by weight of lamivudine, or a pharmaceutically acceptable derivative thereof;
(b) from 20 to 70% by weight of a bulking agent;
(c) from 20 to 50% by weight of a component which is both a bulking agent and a binder; and
(d) from 1 to 20% by weight of a binder.

6. Granules as claimed in claim 5 comprising;
(a') from 1 to 50% by weight of lamivudine, or a pharmaceutically acceptable derivative thereof;
(b-1') from 20 to 50% by weight of lactose;
(b-2') from 1 to 20% by weight of crystalline cellulose;
(c') from 20 to 50% by weight of starch; and
(d') from 1 to 20% by weight of hydroxypropyl cellulose

7. A process for the preparation of a pharmaceutical formulation as claimed in any one of claims 1 to 4, comprising at least two steps
(i) aqueous granulation of components (a) to (d) of the formulation and
(ii) blending the granules formed by step (i) with component (e), of the formulation.

8. A process for the preparation of granules as claimed in claim 5 or claim 6, comprising aqueous granulation of components (a) to (d) of the formulation.

9. Use of lamivudine in the manufacture of a pharmaceutical formulation as claimed in any one of claims 1 to 4 or granules as claimed in any one of claims 5 to 6 for the treatment of viral disease.

10. Use of a pharmaceutical formulation as claimed in any one of claims 1 to 4 or granules as claimed in any one of claims 5 to 6 in the manufacture of a medicament for use in the treatment of a viral infection.

## Patentansprüche

1. Pharmazeutische Formulierung, umfassend:
(a) 1 bis 50 Gew.-% Lamivudin oder eines pharmazeutisch akzeptablen Derivats davon;
(b) 20 bis 70 Gew.-% eines Füllstoffs;
(c) 20 bis 50 Gew.-% einer Komponente, die sowohl ein Füllstoff als auch ein Bindemittel ist;
(d) 1 bis 20 Gew.-% eines Bindemittels; und
(e) 1 bis 10 Gew.-% eines Schmiermittels.

2. Pharmazeutische Formulierung gemäß Anspruch 1, umfassend:
(a') 1 bis 50 Gew.-% Lamivudin oder eines pharmazeutisch akzeptablen Derivats davon;
(b-1') 20 bis 50 Gew.-% Lactose;
(b-2') 1 bis 20 Gew.-% kristalline Cellulose;
(c') 20 bis 50 Gew.-% Stärke;
(d') 1 bis 20 Gew.-% Hydroxypropylcellulose; und
(e') 1 bis 10 Gew.-% Talkum.

3. Pharmazeutische Formulierung gemäß Anspruch 1 oder Anspruch 2 umfassend:
(a") 5 bis 15 Gew.-% Lamivudin oder eines pharmazeutisch akzeptablen Derivats;
(b-1") 32 bis 42 Gew.-% Lactose;
(b-2") 1 bis 10 Gew.-% kristalline Cellulose;
(c") 32 bis 42 Gew.-% Stärke;
(d") 3 bis 13 Gew.-% Hydroxypropylcellulose; und
(e") 1 bis 5 Gew.-% Talkum.

4. Pharmazeutisch akzeptable Formulierung, die 10 Gew.-% Lamivudin, 37 Gew.-% von jeweils Lactose und Stärke, 5 Gew.-% kristalline Cellulose, 8 Gew.-% Hydroxypropylcellulose und 3 Gew.-% Talkum umfaßt.

5. Granalien, umfassend:
(a) 1 bis 50 Gew.-% Lamivudin oder eines pharmazeutisch akzeptablen Derivats davon;
(b) 20 bis 70 Gew.-% eines Füllstoffs;
(c) 20 bis 50 Gew.-% einer Komponente, die sowohl ein Füllstoff als auch ein Bindemittel ist; und
(d) 1 bis 20 Gew.-% eines Bindemittels.

6. Granalien gemäß Anspruch 5, umfassend:
(a') 1 bis 50 Gew.-% Lamivudin oder eines pharmazeutisch akzeptablen Derivats davon;
(b-1') 20 bis 50 Gew.-% Lactose;
(b-2') 1 bis 20 Gew.-% kristalline Cellulose;
(c') 20 bis 50 Gew.-% Stärke;
(d') 1 bis 20 Gew.-% Hydroxypropylcellulose.

7. Verfahren zur Herstellung einer pharmazeutischen Formulierung gemäß einem der Ansprüche 1 bis 4, umfassend wenigstens zwei Schritte:
(i) wäßrige Granulierung der Komponenten (a) bis (d) der Formulierung und
(ii) Vermischen der durch Schritt (i) gebildeten Granalien mit Komponente (e) der Formulierung.

8. Verfahren zur Herstellung von Granalien gemäß Anspruch 5 oder Anspruch 6, umfassend die wäßrige Granulierung der Komponenten (a) bis (d) der Formulierung.

9. Verwendung von Lamivudin in der Herstellung einer pharmazeutischen Formulierung gemäß einem der Ansprüche 1 bis 4 oder von Granalien gemäß einem der Ansprüche 5 bis 6 zur Behandlung einer viralen Erkrankung.

10. Verwendung einer pharmazeutischen Formulierung gemäß einem der Ansprüche 1 bis 4 oder von Granalien gemäß einem der Ansprüche 5 bis 6 in der Herstellung eines Medikaments zur Verwendung in der Behandlung einer viralen Infektion.

## Revendications

1. Formulation pharmaceutique comprenant :
(a) de 1 à 50% en poids de lamivudine ou d'un dérivé de celle-ci acceptable du point de vue pharmaceutique;
(b) de 20 à 70% en poids d'un diluant ;
(c) de 20 à 50% en poids d'un composant qui est à la fois un diluant et un liant ;
(d) de 1 à 20% en poids d'un liant ; et
(e) de 1 à 10% en poids d'un lubrifiant.

2. Formulation pharmaceutique selon la revendication 1, comprenant :
(a') de 1 à 50% en poids de lamivudine ou d'un dérivé de celle-ci acceptable du point de vue pharmaceutique ;
(b-1') de 20 à 50% en poids de lactose ;
(b-2') de 1 à 20% en poids de cellulose cristalline ;
(c') de 20 à 50% en poids d'amidon ;
(d') de 1 à 20% en poids d'hydroxypropylcellulose ; et
(e') de 1 à 10% en poids de talc.

3. Formulation pharmaceutique selon la revendication 1 ou la revendication 2, comprenant :
(a'') de 5 à 15% en poids de lamivudine ou d'un dérivé de celle-ci acceptable du point de vue pharmaceutique ;
(b-1'') de 32 à 42% en poids de lactose ;
(b-2'') de 1 à 10% en poids de cellulose cristalline ;
(c'') de 32 à 42% en poids d'amidon ;
(d'') de 3 à 13% en poids d'hydroxypropylcellulose ; et
(e'') de 1 à 5% en poids de talc.

4. Formulation acceptable du point de vue pharmaceutique comprenant 10% en poids de lamivudine, 37% en poids chacun de lactose et d'amidon, 5% en poids de cellulose cristalline, 8% en poids d'hydroxypropyl cellulose et 3% en poids de talc.

5. Granulés comprenant :
(a) de 1 à 50% en poids de lamivudine ou d'un dérivé de celle-ci acceptable du point de vue pharmaceutique ;
(b) de 20 à 70% en poids d'un diluant ;
(c) de 20 à 50% en poids d'un composant qui est à la fois un diluant et un liant ; et
(d) de 1 à 20% en poids d'un liant.

6. Granulés selon la revendication 5, comprenant :
(a') de 1 à 50% en poids de lamivudine ou d'un dérivé de celle-ci acceptable du point de vue pharmaceutique ;
(b-1') de 20 à 50% en poids de lactose ;
(b-2') de 1 à 20% en poids de cellulose cristalline ;
(c') de 20 à 50% en poids d'amidon ; et
(d') de 1 à 20% en poids d'hydroxypropylcellulose.

7. Procédé pour la préparation d'une formulation pharmaceutique selon l'une quelconque des revendications 1 à 4, comprenant au moins deux étapes :
(i) granulation aqueuse des composants (a) à (d) de la formulation et
(ii) mélange des granulés formés par l'étape (i) avec le composant (e) de la formulation.

8. Procédé pour la préparation de granulés selon la revendication 5 ou la revendication 6, comprenant la granulation aqueuse des composants (a) à (d) de la formulation.

9. Utilisation de lamivudine dans la fabrication d'une formulation pharmaceutique selon l'une quelconque des revendications 1 à 4 ou de granulés selon l'une quelconque des revendications 5 à 6 pour le traitement d'affection virale.

10. Utilisation d'une formulation pharmaceutique selon l'une quelconque des revendications 1 à 4 ou de granulés selon l'une quelconque des revendications 5 à 6 dans la fabrication d'un médicament utile dans le traitement d'une affection virale.
